(19) Europäisches Patentamt
European Patent Office
Office européen des brevets

(11) Veröffentlichungsnummer: **0 324 181 B1**

# (12) EUROPÄISCHE PATENTSCHRIFT

(45) Veröffentlichungstag der Patentschrift: **27.11.91**

(51) Int. Cl.5: **C07D 281/08**, C07D 281/10

(21) Anmeldenummer: **88121879.6**

(22) Anmeldetag: **30.12.88**

Die Akte enthält technische Angaben, die nach dem Eingang der Anmeldung eingereicht wurden und die nicht in dieser Patentschrift enthalten sind.

## (54) Verfahren zur Herstellung von Benzothiazepinon-Derivaten.

(30) Priorität: **09.01.88 DE 3800386**

(43) Veröffentlichungstag der Anmeldung: **19.07.89 Patentblatt 89/29**

(45) Bekanntmachung des Hinweises auf die Patenterteilung: **27.11.91 Patentblatt 91/48**

(84) Benannte Vertragsstaaten: **BE CH DE FR GB IT LI**

(56) Entgegenhaltungen:
**DE-C- 823 738**
**FR-A- 2 141 777**
**US-A- 2 791 612**
**US-A- 3 075 967**

**BULLETIN DES SOCIETES CHIMIQUES BELGES, Band 60, 1951, Seiten 319-324, Osford, GB; J. NYS et al.: "Synthèse et caractérisation de l'o-amino-thiophénol"**

(73) Patentinhaber: **BAYER AG**

**W-5090 Leverkusen 1 Bayerwerk(DE)**

(72) Erfinder: **Mais, Franz-Josef, Dr.**
**Gustav-Poensgen-Strasse 23**
**W-4000 Düsseldorf 1(DE)**
Erfinder: **Fiege, Helmut, Dr.**
**Walter-Flex-Strasse 23**
**W-5090 Leverkusen 1(DE)**

EP 0 324 181 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Verfahren zur Herstellung von Benzothiazepinon-Derivaten aus gegebenenfalls substituierten Benzothiazolen, die zunächst zu o-Amino-thiophenolen verseift werden und danach mit gegebenenfalls substituierten Acrylsäuren weiter umgesetzt werden.

Benzothiazepinon-Derivate sind hervorragend geeignete Co-Katalysatoren zur Steuerung von Isomeren-Verhältnissen bei Kernchlorierungen von Alkylbenzolen in Gegenwart von Friedel-Crafts-Katalysatoren.

Aus US 2.791.612 ist bekannt, daß die Herstellung von o-Amino-thiophenol durch alkalische Hydrolyse von Benzothiazol und anschließendes Ansäuern mit Mineralsäure zu Nebenprodukten führt, die teilweise die Rückreaktion zu Benzothiazol eingehen; auch bei sorgfältiger Einhaltung der Reaktionsparameter werden daher nur unbefriedigende Ausbeuten erhalten. Daher wird vorgeschlagen, mit Essigsäure statt mit einer Mineralsäure zu arbeiten.

Aus US 3.075.967 ist die Umsetzung von gegebenenfalls substituierten o-Amino-thiophenolen mit gegebenenfalls substituierten Acrylsäuren bekannt. Beispielsweise werden Phenylcrotonsäure und o-Amino-thiophenol bei 160-175 °C zusammengeschmolzen. Nachteilig hierbei ist, daß diese Umsetzungen in der Schmelze zu niedrigen Ausbeuten führen (J. Chem. Soc. 130 (1927), 2738) und daß hierbei nicht nur Benzothiazepinon-Derivate sondern auch isomere Benzothiazin-Derivate gebildet werden (Chem. Ber. 119 (1986), 3109).

Weiterhin ist die Umsetzung einiger substituierter Acrylsäuren mit o-Amino-thiophenol in hochsiedenden, mit Wasser mischbaren Lösungsmitteln bekannt (Tetrahedron 42 (1986), 2731); die Benzothiazepinon-Derivate sind hierbei nur durch wäßrige Aufarbeitung isolierbar.

Es wurde nun ein Verfahren zur Herstellung von Benzothiazepinon-Derivaten der Formel

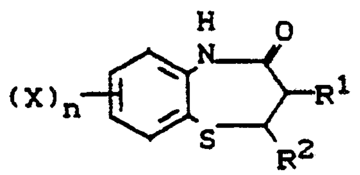

(I),

worin

| | |
|---|---|
| $R^1$ und $R^2$ | unabhängig voneinander für Wasserstoff, $C_1$-$C_4$-Alkyl oder $(X)_n$-Phenyl stehen oder gemeinsam $C_3$-$C_5$-Alkylen bilden, |
| X | für Wasserstoff, $C_1$-$C_4$-Alkyl oder $C_1$-$C_4$-Alkoxy steht und |
| n | 1 oder 2 |

bedeutet,
durch alkalische Verseifung von Benzothiazolen der Formel

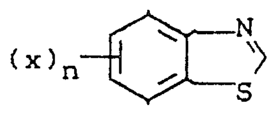

(II),

zu o-Amino-thiophenolen der Formel

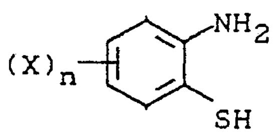

(III),

deren Isolierung durch Ansäuern und Umsetzung mit Acrylsäuren der Formel

$R^2CH = CR^1$-COOH (IV),

worin $R^1$, $R^2$, X und n die genannte Bedeutung haben, gefunden, das dadurch gekennzeichnet ist, daß man das Ansäuern mit einer Mineralsäure vornimmt, danach mit einem wasserunlöslichen Lösungsmittel

extrahiert und den Extrakt ohne Entfernung des Lösungsmittels mit der Acrylsäure umsetzt.

Die Reste $R^1$ und $R^2$ bedeuten unabhängig voneinander Wasserstoff, $C_1$-$C_4$-Alkyl, wie Methyl, Ethyl, Propyl, Isopropyl, Butyl oder Isobutyl, oder Phenyl, welches ein-oder zweimal mit dem weiter unten beschriebenen Substituenten X substituiert ist, sowie weiterhin gemeinsam $C_3$-$C_5$-Alkylen, wie Trimethylen, Tetramethylen oder Pentamethylen. In bevorzugter Weise bedeuten $R^1$ und $R^2$ unabhängig voneinander Wasserstoff, Methyl oder Phenyl, sowie gemeinsam die Tetramethylen-Gruppe. In besonders bevorzugter Weise ist einer der Reste $R^1$ und $R^2$ Wasserstoff und der andere Wasserstoff, Methyl oder Phenyl. Der Substituent X steht für Wasserstoff, $C_1$-$C_4$-Alkyl, wie die oben genannten, oder $C_1$-$C_4$-Alkoxy, wie Methoxy, Ethoxy, Propoxy, Isopropoxy, Butoxy oder Isobutoxy. In bevorzugter Weise steht der Substituent X für Wasserstoff, Methyl oder Methoxy, in besonders bevorzugter Weise für Wasserstoff. Der Index n steht für die Zahl 1 oder 2, bevorzugt für die Zahl 1.

Als Beispiel für die erfindungsgemäß herstellbaren Verbindungen der Formel (I) seien namentlich genannt, wobei das System wie folgt numeriert wurde:

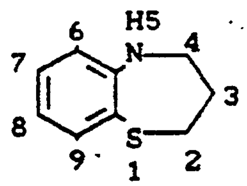

2,3-Dihydro-1,5-benzothiazepin-4(5H)-on,
2,3-Dihydro-2-methyl-1,5-benzothiazepin-4(5H)-on,
2,3-Dihydro-3-methyl-1,5-benzothiazepin-4(5H)-on,
2,3-Dihydro-7,8-dimethyl-1,5-benzothiazepin-4-(5H)-on,
2,3-Dihydro-7-methyl-1,5-benzothiazepin-4(5H)-on,
2,3-Dihydro-2,3-dimethyl-1,5-benzothiazepin-4(5H)-on,
2,3-Dihydro-2,3-tetramethylen-1,5-benzothiazepin-4(5H)-on,
2,3-Dihydro-2-phenyl-1,5-benzothiazepin-4(5H)-on,
2,3-Dihydro-7-methoxy-1,5-benzothiazepin-4(5H)-on.

Die Verseifung der Benzothiazole der Formel (II) erfolgt mit wäßrigen alkalischen Lösungen. Im allgemeinen kann dazu jede Verbindung eingesetzt werden, die eine genügend hohe OH-Ionen-Konzentration in wäßriger Lösung erzeugt. Bevorzugt werden Lösungen von Alkalihydroxiden, beispielsweise mit einer Konzentration von 20 bis 60 Gew.-%, bevorzugt 30 bis 50 Gew.-%, besonders bevorzugt 30 bis 50 Gew.-% NaOH-Lösung, eingesetzt.

Die Hydrolyse läuft in einem weiten Temperaturbereich bis zum Siedepunkt der Mischung aus der wäßrigen alkalischen Lösung und Benzothiazolen der Formel (II) ab. Tiefe Temperaturen im Bereich der Raumtemperatur sind im allgemeinen wegen der geringen Reaktionsgeschwindigkeit weniger vorteilhaft. Bevorzugt sind daher Temperaturen von 50 °C, bevorzugt von 100 °C, bis zum Siedepunkt des Hydrolysegemisches; besonders bevorzugt ist das Arbeiten bei Rückflußtemperatur.

Die alkalische Verseifungsmischung muß zur Freisetzung der o-Amino-thiophenole der Formel (III) angesäuert werden. Zum Ansäuern ist erfindungsgemäß jede Mineralsäure geeignet, beispielsweise Salpetersäure, Schwefelsäure, Salzsäure oder Phosphorsäure, mit oder ohne Verdünnung mit Wasser. Bevorzugt ist die wäßrig verdünnte Form. Besonders bevorzugt ist eine 30 bis 70 gew.-%ige Schwefelsäure oder eine 20 bis 35 gew.-%ige Salzsäure. Die Konzentration der Säure ist aus praktischen Gründen vorteilhaft so zu wählen, daß die entstehenden Salze nicht aus der wäßrigen Phase ausfallen und die anschließende Phasentrennung erschweren. Gegebenenfalls kann die Mischung mit weiterem Wasser in Abhängigkeit von der jeweiligen Temperatur verdünnt werden, bis etwa ausgefallene Salze wieder gelöst sind.

Die Temperatur, bei der die Neutralisation mit der Mineralsäure durchgeführt wird, ist im allgemeinen bis zum Siedepunkt der Mischung beliebig. Bevorzugt wird die Neutralisation in einem Temperaturbereich von 60 bis 120 °C, besonders bevorzugt bei 80 bis 100 °C, ausgeführt.

Das sich beim Ansäuern bildende o-Amino-thiophenol der Formel (III) wird durch Zugabe eines Lösungsmittels extrahiert. Hierzu ist prinzipiell jedes wasserunlösliche Lösungsmittel geeignet. Beispiele hierfür sind aliphatische Kohlenwasserstoffe, aliphatische Halogen-Kohlenwasserstoffe, aromatische Kohlenwasserstoffe, aromatische Halogen-Kohlenwasserstoffe, wasserunlösliche Ester oder wasserunlösliche Ether; in bevorzugter Weise werden aromatische Kohlenwasserstoffe und aromatische Halogen-Kohlenwasserstoffe, in besonders bevorzugter Weise aromatische Halogen-Kohlenwasserstoffe, eingesetzt. Beispiel für

solche besonders bevorzugten aromatischen Halogen-Kohlenwasserstoffe sind: Chlorbenzol, Brombenzol, die isomeren Dichlorbenzole, die isomeren Chlortoluole oder die isomeren Bromtoluole.

Die Umsetzung des Extrakts des o-Amino-thiophenols der Formel (III) mit der Acrylsäure der Formel (IV) kann ohne weitere Reinigung dieses Extrakts vorgenommen werden. Sie erfolgt in einem weiten Temperaturbereich von Raumtemperatur bis zum Siedepunkt des Reaktionsgemisches. Es ist bevorzugt, die Umsetzung zunächst in einem Bereich von 80 bis 120 °C zu führen und dann gegebenenfalls die Temperatur bis zum Siedepunkt des Reaktionsgemisches zu erhöhen. In einer bevorzugten Variante wird hierbei das Reaktionswasser durch azeotrope Destillation mit dem Extraktionslösungsmittel aus dem Reaktionsgemisch ausgeschleust, wobei in einer bekannten Weise das hierbei mitdestillierte Extraktionsmittel nach der Abtrennung im Wasserabscheider wieder in das Reaktionsgemisch zurückgeführt wird. Hierzu kann vorteilhafterweise das Reaktionsgemisch solange under Rückfluß erhitzt werden, bis sich aus dem Kondensat kein Wasser mehr abscheidet.

Beim Abkühlen des Reaktionsgemisches scheidet sich das Benzothiazepinon-Derivat der Formel (I) ab. Vorteilhafterweise wird hierzu bis auf Raumtemperatur gekühlt, jedoch ist auch ein Arbeiten bei leicht erhöhter Temperatur möglich. Dabei fällt das Reaktionsprodukt der Formel (I) bereits in reiner Form aus und kann durch übliche Methoden, beispielsweise durch Filtration oder Zentrifugieren, von der Mutterlauge getrennt werden. Falls erforderlich, kann das abgetrennte Reaktionsprodukt (I) mit dem Extraktionslösungsmittel, das auch Reaktionsmedium war, gewaschen werden.

Die Benzothiazepinon-Derivate der Formel (I) sind hervorragende Co-Katalysatoren neben den üblichen Friedel-Crafts-Katalysatoren zur Kernchlorierung von Alkylbenzolen. Diese Co-Katalysate erlaubt die Regelung des ortho-/para-Verhältnisses. Hierzu können die Benzothiazepinon-Derivate in der lösungsmittelfeuchten Form oder auch nach vorangegangener Trocknung eingesetzt werden.

Die Gewinnung und der Einsatz der Benzothiazepinon-Derivate in lösungsmittelfeuchter Form ist die bevorzugte und technisch günstigste Variante. Das jeweils gewählte Extraktionslösungsmittel/Reaktionsmedium ergibt sich aus der angestrebten Verwendung der Benzothiazepinone: Sollen sie z.B. für eine Co-Katalyse der Toluol-Kernchlorierung eingesetzt werden, ist das besonders bevorzugte Lösungsmittel zur Extraktion (späteres Reaktionsmedium) Toluol, ein Chlortoluol oder ein Chlortoluolgemisch, bevorzugt ein Chlortoluolgemisch. Will man die Benzothiazepinone jedoch beispielsweise für die Co-Katalyse von Cumol-Kernchlorierungen einsetzen, wird Cumol, Chlorcumol und/oder ein Chlorcumolgemisch, bevorzugt ein Chlorcumolgemisch als Extraktionslösungsmittel verwendet.

Es ist überraschend, daß nach dem erfindungsgemäßen Verfahren im Gegensatz zur Lehre der US 2.791.612 durch Ansäuern mit Mineralsäure die o-Amino-thiophenole in reiner Form und hoher Ausbeute isoliert werden können. Ebenso überraschend ist die Steigerung der Ausbeuten an den Benzothiazepinon-Derivaten durch Verwendung der genannten Extraktionslösungsmittel. Die Einfachheit der Isolierung der Benzothiazepinon-Derivate aus den Mutterlaugen ist technisch sehr vorteilhaft. Auch der Einsatz der Benzothiazepinon-Derivate ohne vorangegangene Trocknung als Co-Katalysatoren bei der Kernchlorierung von Alkylaromaten ist ein Vorteil des erfindungsgemäßen Verfahrens.

Die nachfolgenden Beispiele sollen das erfindungsgemäße Verfahren verdeutlichen, ohne es jedoch auf diese Beispiele einzuschränken.

Beispiel 1

2,3-Dihydro-1,5-benzothiazepin-4(5H)-on

Man legte 100 Gew.-Teile 50 %-NaOH-Lösung vor und erhitzte zum Rückfluß. In 3 Stunden wurden 66 Gew.-Teile Benzothiazol zugefügt und dann 2 h unter Rückfluß nachgerührt. Man kühlte etwas ab und säuerte bei 100 °C mit 120 Gew.-Teilen konz. HCl an und gab dann noch 100 Gew.-Teile Wasser dazu. Man versetzte mit 70 Gew.-Teilen einer Chlortoluolmischung aus ca. 50 % o-Chlortoluol und ca. 50 % p-Chlortoluol und trennte die sich scheidende Wasserphase ab. Die organische Phase erhitzte man auf 100 °C, fügte innerhalb 1 Stunde 35 Gew.-Teile Acrylsäure zu und erhitzte solange zum Rückfluß, bis sich aus dem Kondensat kein Wasser mehr abschied. Die organische Phase des Kondensats wurde in das Reaktionsgemisch zurückgeführt. Dann kühlte man auf ca. 25 ° C ab und saugte den Feststoff ab. Man wusch mit 70 Gew.-Teilen des Chlortoluolgemisches.

Zur Bestimmung der Ausbeute wurde getrocknet.

Auswaage: 71 Gew.-Teile = 81 % der Theorie;
Schmelzpunkt: 216 - 218 ° C.

Beispiel 2

2,3-Dihydro-2-methyl-1,5-benzothiazepin-4(5H)-on

Man verfuhr wie in Beispiel 1, nur wurden statt der Acrylsäure hier 42 Gew.-Teile Methacrylsäure zugeführt.

Nach dem Trocknen erhielt man eine Auswaage von 61,5 Gew.-Teilen = 65 % der Theorie; Schmelzpunkt: 176 - 177° C.

Beispiel 3

2,3-Dihydro-3-methyl-1,5-benzothiazepin-4(5H)-on

Man verfuhr zunächst wie in Beispiel 1. Dann säuerte man bei 100 bis 110° C mit 70 Gew.-Teilen ca. 50 %iger Schwefelsäure an und gab 150 Gew.-Teile Wasser und dann 70 Gew.-Teile des Chlortoluolgemisches zu. Nach dem Abtrennen der wäßrigen Phase wurde die organische Phase auf 100° C erhitzt und innerhalb 1 h mit 42 Gew.-Teilen flüssiger Crotonsäure versetzt. Nach der Zugabe wurde bis zum Ende der Wasserabscheidung unter Rückfluß erhitzt, dann auf ca. 30° C gekühlt, abgesaugt und mit 80 Gew.-Teilen des Chlortoluolgemisches gewaschen.

Zur Bestimmung der Ausbeute wurde getrocknet.

Auswaage: 85,5 Gew.-Teile = 90,5 % der Theorie;
Schmelzpunkt: 206 - 207° C.

Beispiel 4

2,3-Dihydro-2-phenyl-1,5-benzothiazepin-4(5H)-on

Man verfuhr wie in Beispiel 3, nur wurden statt der Crotonsäure 72 Gew.-Teile Zimtsäure, gemischt mit 72 Gew.-Teilen Chlortoluolmischung, in flüssiger Form zugeführt.

Nach dem Trocknen erhielt man eine Auswaage von 96 Gew.-Teilen = 77 % der Theorie; Schmelzpunkt: 175 - 176° C.

Beispiele 5-7

In analoger Weise wie in den vorausgegangenen Beispielen erhält man aus Benzothiazol und 2-Hexensäure das 2,3-Dihydro-3-propyl-1,5-benzothiazepin-4(5H)-on (Schmp.: 120-122° C), aus Benzothiazol und Cyclohexen-1-carbonsäure das 2,3-Dihydro-2,3-tetramethylen-1,5-benzothiazepin-4(5H)-on (Schmp.: 225-227° C) und aus 5,6-Dimethylbenzothiazol und Acrylsäure das 2,3-Dihydro-7,8-dimethyl-1,5-benzothiazepin-4(5H)on (Schmp.: 241-244° C).

Verwendungsbeispiel 8

Es wurde eine Lösung aus 100 Gew.-Teilen Toluol, 0,0175 Gew.-Teilen $FeCl_3$ und 0,004 Gew.-Teilen der Verbindung der Formel

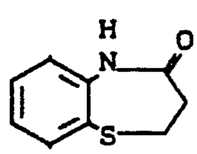

(2,3-Dihydro-1,5-benzothiazepin-4(5)-on)

hergestellt. Diese Lösung führte man einem kontinuierlich arbeitenden Chlorierreaktor bei 40-43° C zu, wobei gleichzeitig die äquivalente Menge an Chloriergemisch entnommen wurde. Gasförmiges Chlor wurde so schnell zugeführt, daß der Umsatz etwa konstant bei 90 mol-% lag. Das abgeführte Reaktionsgemisch enthielt ca. 7,1 Gew.-% Toluol, das Verhältnis ortho-Chlortoluol zu para-Chlortoluol war o/p = 0,70.

Verwendungsbeispiel 9

Das Verfahren des Beispiels 8 wurde wiederholt. Man gab jedoch 0,005 Gew.-Teile der Verbindung der

Formel

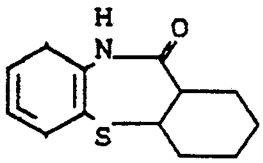

(2,3-Dihydro-2,3-tetramethylen-1,5-benzothiazepin-4(5H)-on)

zu. Das abgekühlte Reaktionsgemisch enthielt ca. 7,0 Gew.-% Toluol, das Verhältnis ortho-Chlortoluol zu para-Chlortoluol war o/p = 0,64.

## Patentansprüche

**1.** Verfahren zur Herstellung von Benzothiazepinon-Derivaten der Formel

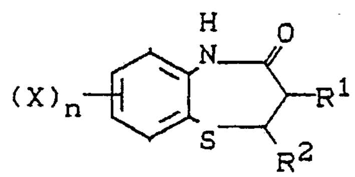

,

worin

| | |
|---|---|
| $R^1$ und $R^2$ | unabhängig voneinander für Wasserstoff, $C_1$-$C_4$-Alkyl oder $(X)_n$-Phenyl stehen oder gemeinsam $C_3$-$C_5$-Alkylen bilden, |
| X | für Wasserstoff, $C_1$-$C_4$-Alkyl oder $C_1$-$C_4$-Alkoxy steht und |
| n | 1 oder 2 |

bedeutet,

durch alkalische Verseifung von Benzothiazolen der Formel

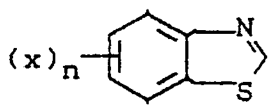

,

zu o-Amino-thiophenolen der Formel

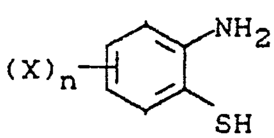

,

deren Isolierung durch Ansäuern und Umsetzung mit Acrylsäuren der Formel

$R^2CH = CR^1\text{-}COOH$ ,

wobei $R^1$, $R^2$, X und n die genannte Bedeutung haben,

dadurch gekennzeichnet, daß man das Ansäuern mit einer Mineralsäure vornimmt, danach mit einem wasserunlöslichen Lösungsmittel extrahiert und den Extrakt ohne Entfernung des Lösungsmittels mit der Acrylsäure umsetzt.

2. Verfahren nach Anspruch 1, dadurch gekennzeichnet, daß als Mineralsäure Salpetersäure, Schwefelsäure, Salzsäure oder Phosphorsäure mit oder ohne Verdünnung mit Wasser eingesetzt werden.

3. Verfahren nach Anspruch 2, dadurch gekennzeichnet, daß wäßrig verdünnte Mineralsäuren eingesetzt werden.

4. Verfahren nach Anspruch 1, dadurch gekennzeichnet, daß als Extraktionslösungsmittel aromatische Kohlenwasserstoffe und aromatische Halogen-Kohlenwasserstoffe eingesetzt werden.

5. Verfahren nach Anspruch 1, dadurch gekennzeichnet, daß als Extraktionslösungsmittel aromatische Halogen-Kohlenwasserstoffe eingesetzt werden.

6. Verfahren nach Anspruch 4, dadurch gekennzeichnet, daß zur späteren Verwendung der Benzothiazepinon-Derivate bei der Toluol-Kernchlorierung als Extraktionslösungsmittel Chlortoluol eingesetzt wird.

7. Verfahren nach Anspruch 1, dadurch gekennzeichnet, daß die Umsetzung der o-Amino-thiophenole mit den Acrylsäuren unter Auschleusen von Wasser durchgeführt wird.

8. Verfahren nach Anspruch 6, dadurch gekennzeichnet, daß die Umsetzungstemperatur bis zur Siedetemperatur des Umsetzungsgemisches erhöht wird.

9. Verfahren nach Anspruch 1, dadurch gekennzeichnet, daß die Benzothiazepinon-Derivate durch Kristallisation aus der Reaktionslösung isoliert werden.

10. Verfahren nach Anspruch 8, dadurch gekennzeichnet, daß die Benzothiazepinon-Derivate als lösungsmittelfeuchtes Kristallisat isoliert werden.

11. Verfahren nach Anspruch 1, dadurch gekennzeichnet, daß als Benzothiazepinon-Derivat das 2,3-Dihydro-1,5-benzothiazepin-4(5H)-on erhalten wird.

**Claims**

1. Process for the preparation of benzothiazepinone derivatives of the formula

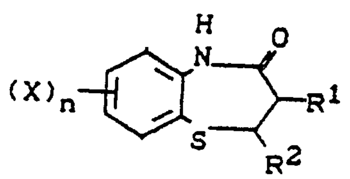,

wherein

| | |
|---|---|
| $R^1$ and $R^2$ | independently of one another represent hydrogen, $C_1$-$C_4$-alkyl or $(X)_n$-phenyl or together form $C_3$-$C_5$-alkylene, |
| X | represents hydrogen, $C_1$-$C_4$-alkyl or $C_1$-$C_4$-alkoxy and |
| n | denotes 1 or 2, |

by alkaline hydrolysis of benzothiazoles of the formula

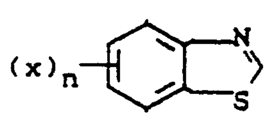,

to give o-amino-thiophenols of the formula

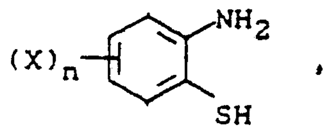

isolation thereof by acidification and reaction with acrylic acids of the formula

$R^2CH = CR^1\text{-}COOH$

wherein $R^1$, $R^2$, X and n have the meaning mentioned,

characterised in that the acidification is carried out using a mineral acid, the mixture is then extracted using a water-insoluble solvent and the extract is reacted with the acrylic acid without removal of the solvent.

2. Process according to Claim 1, characterised in that nitric acid, sulphuric acid, hydrochloric acid or phosphoric acid, with or without dilution with water are employed as the mineral acid.

3. Process according to Claim 2, characterised in that aqueous dilute mineral acids are employed.

4. Process according to Claim 1, characterised in that aromatic hydrocarbons and aromatic halogenated hydrocarbons are employed as the extracting solvent.

5. Process according to Claim 1, characterised in that aromatic halogenated hydrocarbons are employed as the extracting solvent.

6. Process according to Claim 4, characterised in that, for later use of the benzothiazepinone derivatives in the nuclear chlorination of toluene, chlorotoluene is employed as the extracting solvent.

7. Process according to Claim 1, characterised in that the reaction of the o-amino-thiophenols with the acrylic acids is carried out with the exclusion of water.

8. Process according to Claim 6, characterised in that the reaction temperature is increased up to the boiling temperature of the reaction mixture.

9. Process according to Claim 1, characterised in that the benzothiazepinone derivatives are isolated from the reaction solution by crystallisation.

10. Process according to Claim 8, characterised in that the benzothiazepinone derivatives are isolated as a solvent-moist crystallisate.

11. Process according to Claim 1, characterised in that 2,3-dihydro-1,5-benzothiazepin-4(5H)-one is obtained as benzothiazepinone derivative.

## Revendications

1. Procédé de fabrication de dérivés de benzothiazépinone de formule

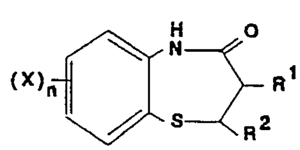

dans laquelle

$R^1$ et $R^2$ désignent, indépendamment l'un de l'autre, l'hydrogène, un radical alkyle $C_1$-$C_4$ ou un radical phényle-$(X)_n$ ou forment ensemble un radical alkylène $C_3$-$C_5$,
X représente l'hydrogène, un radical alkyle $C_1$-$C_4$ ou un radical alkoxy $C_1$-$C_4$ et
n signifie 1 ou 2,

par saponification alcaline de benzothiazoles de formule

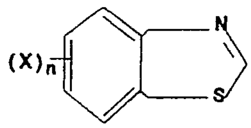

en o-amino-thiophénols de formule

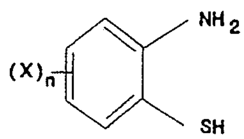

dont l'isolation est effectuée par acidification et réaction avec des acides acryliques de formule

$R^2$-CH = $CR^1$-COOH,

$R^1$, $R^2$, X et n ayant la signification susmentionnée,

caractérisé en ce que l'acidification est accomplie avec un acide minéral, en ce que l'extraction subséquente s'opère avec un solvant non hydrosoluble et en ce que l'extrait réagit avec l'acide acrylique sans élimination du solvant.

2. Procédé selon la revendication 1, caractérisé en ce que l'acide minéral utilisé peut être de l'acide nitrique, de l'acide sulfurique, de l'acide chlorhydrique ou de l'acide phosphorique, dilué ou non dans de l'eau.

3. Procédé selon la revendication 2, caractérisé en ce que des acides minéraux dilués dans de l'eau sont utilisés.

4. Procédé selon la revendication 1, caractérisé en ce que des hydrocarbures aromatiques et des hydrocarbures halogénés aromatiques sont utilisés comme solvant d'extraction.

5. Procédé selon la revendication 1, caractérisé en ce que des hydrocarbures halogénés aromatiques sont utilisés comme solvant d'extraction.

6. Procédé selon la revendication 4, caractérisé en ce que le chlorotoluène est utilisé comme solvant d'extraction en vue de l'utilisation ultérieure des dérivés de benzothiazépinone pour le chlorage de noyaux de toluène.

7. Procédé selon la revendication 1, caractérisé en ce que la réaction des o-amino-thiophénols avec les acides acryliques est effectuée en séparant l'eau.

8. Procédé selon la revendication 6, caractérisé en ce que la réaction de réaction est élevée jusqu'au point d'ébullition du mélange réactif.

**9.** Procédé selon la revendication 1, caractérisé en ce que les dérivés de benzothiazépinone sont isolés de la solution de réaction par cristallisation.

**10.** Procédé selon la revendication 8, caractérisé en ce que les dérivés de benzothiazépinone sont isolés sous forme de produit de cristallisation humide contenant du solvant.

**11.** Procédé selon la revendication 1, caractérisé en ce que le dérivé de benzothiazépinone obtenu est la 2,3-dihydro-1,5-benzothiazépin-4(5H)one.